# EUROPEAN PATENT APPLICATION

(11) **EP 1 122 240 A2**
(43) Date of publication of application: **08.08.2001**
(21) Application number: 01300848.7
(22) Date of filing: 31.01.2001
(51) Int. Cl.: C07C 303/38, C07C 311/48, C07C 303/40

(54) **Process for producing sulfonylimide compound**

(30) Priority: 31.01.2000 JP 2000021578; 04.10.2000 EP 00308751; 17.01.2001 JP 2001008448
(71) Applicant: Morita Chemical Industries Co., Ltd., Osaka-shi, Osaka 531-0072 (JP)
(72) Inventor: Yonezawa, Tetsuo, Yodogawa-ku, Osaka-shi, Osaka 532-0002 (JP); Sakamoto, Yoshitaka, Yodogawa-ku, Osaka-shi, Osaka 532-0002 (JP)
(74) Representative: Brasnett, Adrian Hugh

(57) **Abstract**

The present invention pertains to a process for producing sulfonylimide compounds (represented by the formula (I) MN(SO₂R_{f}¹) (SO₂R_{f}²)) industrially easily at a low cost in an efficient manner, wherein the process comprises reacting at least one of the sulfonyl halogenides represented by the formula (II) R_{f}SO₂X with anhydrous ammonia or an ammonium salt in the presence of a fluorine compound represented by the formula (III) MF, in which: X represents with F or Cl among halogen elements of group VIIb of the periodic table; M represents any one of Li, Na, K and Cs among alkali metals of group Ia in the periodic table; R_{f}¹ and R_{f}², which are the same or different, respectively represent any one of a straight chain or branched compound of a fluoroalkyl, perfluoroalkyl, fluoroallyl or fluoroalkenyl group having 1 to 12 carbon atoms; and R_{f} in the above formula (II) represents the same group as R_{f}¹ or R_{f}² in the formula (I).

## Description

### TECHNICAL FIELD OF THE INVENTION

The present invention relates to a process for producing a sulfonylimide compound represented by the formula (I): MN(SO₂R_{f}¹)(SO₂R_{f}²).

### BACKGROUND

Sulfonylimide compounds are safe as a solute of a battery electrolyte, and battery electrolyte that uses the sulfonylimide compound as a solute has a high energy density and exhibits high conductivity. Hence the sulfonylimide compounds are regarded as a promising solute of a battery electrolyte. Also, the sulfonylimide compounds are useful as a Lewis acid catalyst and an ionic conduction agent.

The sulfonylimide compounds represented by the formula (I) MN(SO₂R_{f}¹) (SO₂R_{f}²) may be synthesized by the process proposed by D.D. Desmarteau et al. in INORGANIC CHEMISTRY, VOL. 23, No. 23, pp. 3720-3723 (1984).

In this synthetic method, as shown by the following formula, trifluoromethylsulfonyl fluoride is reacted with ammonia, the resulting product is treated using hydrochloric acid to produce trifluoromethylsulfonylamide, which is then reacted with sodium methylate and then with hexamethyldisilazane, and the resulting product is reacted with trifluoromethylsulfonyl fluoride, thus obtaining an imide sodium salt.

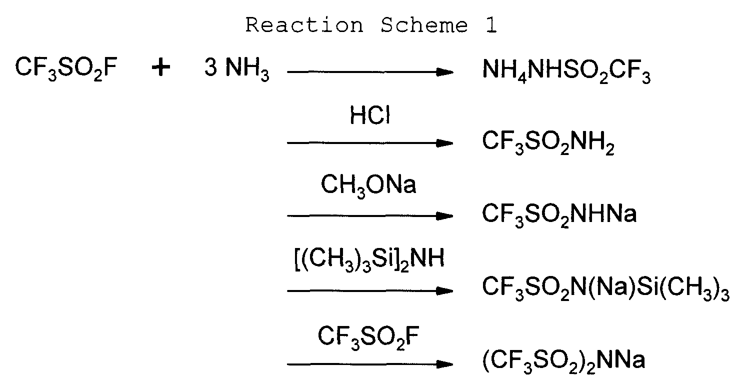

However, this process involves multiple reaction steps and hence takes longer. Also, expensive hexamethyldisilazane must be used to obtain an intermediate, and the yield is as low as about 50%.

In the above described formula (I), M represents any one of Li, Na, K and Cs among alkali metals of group Ia in the periodic table. R_{f}¹ and R_{f}², which may be the same or different, respectively represent any one of a straight chain or branched compound of a fluoroalkyl, perfluoroalkyl, fluoroallyl and fluoroalkenyl group having 1 to 12 carbon atoms (the same hereafter).

In the Japanese Patent Application National Publication No. Hei3-501860, a method is disclosed in which a silazane metal compound is reacted with a perfluorosulfonyl halide compound to obtain an imide compound. In Japanese Patent Application National Publication No. Hei4-501118, a method is disclosed in which an ionic nitride is reacted with a halogenated sulfonic acid to obtain an imide compound.

However, the silazane metal compound and the ionic nitride used in each of the above prior art methods are expensive, and hence the above methods are not an economic production method.

Also, in the Japanese Patent Application Laid-Open (Kokai) No. Hei8-81436, a method is disclosed in which anhydrous ammonia or a sulfonylamide and a sulfonyl fluoride are reacted with a tertiary amine or a heterocyclic amine, and the reaction product is further reacted with, for instance, a hydroxide containing an alkali metal and an alkaline earth metal to produce imide salts.

In this method, because the product in the first stage is generated as an amine salt, it must be further reacted with an inorganic salt. Also, since a tertiary amine or a heterocyclic amine is used in the reaction, problems concerning work environment caused by the odour and disposal of the amine occur. Moreover, because the anhydrous ammonia is always used, an autoclave as the reactor and a low temperature cooling unit are required. This method is therefore unsuitable for mass-production.

As outlined above, the prior art involves a long reaction step and uses expensive raw materials, and it is hence hard to say that these methods in the prior art are industrially acceptable methods.

In the Japanese Patent Application Laid-Open (Kokai) No. Hei8-81436, anhydrous ammonia, a perfluoroalkylsulfonyl fluoride and a tertiary amine are reacted with each other. To obtain an imide salt, at least two steps are required; and in the reaction, a tertiary amine or a heterocyclic amine is used, causing possibility of pollution of work environment derived from the odour and the like. Further, the product must be reacted with an alkali metal or the like in an aqueous solution in the second step, and at this time, it is necessary to dispose the amine which is freed and distilled together with water, causing increased production costs.

One object of the present invention is to solve these various problems and to produce a sulfonylimide compound industrially easily at a low cost in an efficient manner.

### SUMMARY OF THE INVENTION

The inventors of the present invention have found that a sulfonylimide compound (represented by the formula (I) MN(SO₂R_{f}¹) (SO₂R_{f}²)) which is free from the foregoing problems can be produced industrially easily at a low cost in an efficient manner.

Accordingly, one aspect of the present invention pertains to a process for producing a sulfonylamide compound comprising reacting at least one of the sulfonyl halogenides represented by the formula (II) R_{f}SO₂X with anhydrous ammonia or an ammonium salt in the presence of fluorine compounds represented by the formula (III) MF.

In the above-described formulae (I) and (III), M represents any one of Li, Na, K and Cs among alkali metals of group Ia in the periodic table.

In the above-described formula (II), X represents either F or Cl among halogen elements of group VIIb in the periodic table.

In the above-described formula (I), R_{f}¹ and R_{f}², which may be the same or different, respectively represent any one of a straight chain or branched compound of a fluoroalkyl, perfluoroalkyl, fluoroallyl and fluoroalkenyl group having 1 to 12 carbon atoms.

Also, R_{f} in the above-described formula (II) represents the same or identical group as R_{f}¹ or R_{f}² in the formula (I) .

Examples of sulfonylimides include, but are not limited to, the following: LiN(SO₂CF₃)₂, NaN(SO₂CF₃)₂, KN(SO₂CF₃)₂, CsN(SO₂CF₃)₂, LiN(SO₂C₄F₉)₂, NaN(SO₂C₄F₉)₂, KN(SO₂C₄F₉)₂, CsN(SO₂C₄F₉)₂, LiN (SO₂CF₃) (SO₂C₄F₉), NaN (SO₂CF₃) (SO₂C₄F₉), KN(SO₂CF₃)(SO₂C₄F₉), CsN(SO₂CF₃)(SO₂C₂F₅), and CsN(SO₂CF₃)(SO₂C₄F₉).

Another aspect of the present invention pertains to a process for producing a sulfonylamide compound represented by the formula (I) MN(SO₂R_{f}¹) (SO₂R_{f}²) by only one stage reaction, under mild conditions, where anhydrous ammonia is not always used, comprising reacting a sulfonylamide represented by the formula (IV) R_{f}SO₂NH₂, at least one of the sulfonyl halogenides represented by the formula (II) R_{f}SO₂X, and fluorine compound represented by the formula (III) MF with each other.

Li, Na, K, Rb, Cs and Fr exist as the alkali metals of Ia group in the periodic table. Among these metals, especially any one of Li, Na, K and Cs may be selected and the corresponding fluorine compound used accordingly. Therefore, in the case of these metals, the fluorine compounds are LiF, NaF, KF (as KF, any one of calcine-dried KF (cd KF), e.g., produced by a smoking method and spray-dried KF (sd KF), e.g., by a spray drying method may be used) and CsF.

The reason why Li, Na, K and Cs are preferred among alkali metals of group Ia in the periodic table is that they are relatively cheap and suitable to produce sulfonylimide compounds industrially easily, at a low cost and in an efficient manner. Especially, K is prominent for the above property among Li, Na, K and Cs.

In the present invention, on the other hand, the sulfonylimide compound may be produced by using an ammonium salt. As the ammonium salt in this case, it is desirable to use ammonium fluoride or ammonium hydrogendifluoride. The use of either one of these compounds has the advantage that a specific reactor (autoclave) is not required.

CF₃SO₂Cl among sulfonyl halogenides represented by the formula (II) R_{f}SO₂X, which is sold on the market as a reagent, can be usually handled as a liquid because its boiling point is 30°C, which is relatively high among these kinds of compounds.

The terms "sulfonylimide" and "sulfonimide" are used interchangeably herein. Similarly, the terms "sulfonylamide" and "sulfonamide" are used interchangeably herein.

### DETAILED DESCRIPTION OF THE PREFERRED EMBODIMENTS

The embodiments of the invention will be hereinafter explained in detail.

The object compound, which has previously been produced in a multi-step process in the prior art, can be produced in one step by introducing a fluorine compound represented by the formula (III) MF, at least one of the sulfonyl halogenides represented by the formula (II) R_{f}SO₂X, and anhydrous ammonia or an ammonium salt into an inert solvent and reacting the mixture as shown by the following reactions 2, 3, 4 and 5.

This is due to the basicity of the fluorine compound represented by the formula (III) MF.
(1) In the case where R_{f}¹ and R_{f}² in the formula (I) MN(SO₂R_{f}¹)(SO₂R_{f}²) are the same or equal to each other: 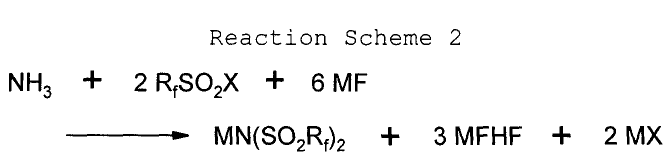 1 mol of anhydrous ammonia, 2 mol of at least one of the sulfonyl halogenides represented by the formula (II) R_{f}SO₂X and 6 mol of a fluorine compound represented by the formula (III) MF are introduced into a reactor and the mixture is reacted in a solvent.
   After completion of the reaction, 2 mol of the by-product MX and 3 moles of hydrogendifluoride salt MFHF are removed by filtration, and the filtrate is concentrated. The sulfonylimide compound represented by the formula (I) MN(SO₂R_{f})₂ can be thereby produced.
(2) In the case where R_{f}¹ and R_{f}² in the formula (I) MN(SO₂R_{f}¹) (SO₂R_{f}²) are different from each other:
   A sulfonylamide containing the R_{f}¹ group which is produced by a known process shown below is reacted with at least one of the sulfonyl halogenides having a desired R_{f}² group. A sulfonylimide compound represented by the formula (I) MN(SO₂R_{f}¹) (SO₂R_{f}²) with the desired R_{f}¹ and R_{f}² groups can be thereby produced. 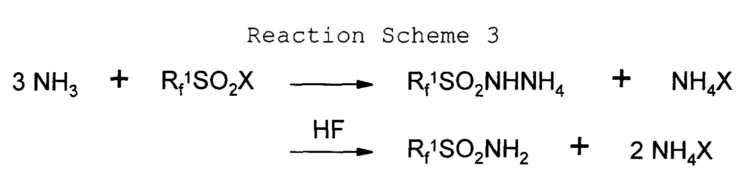 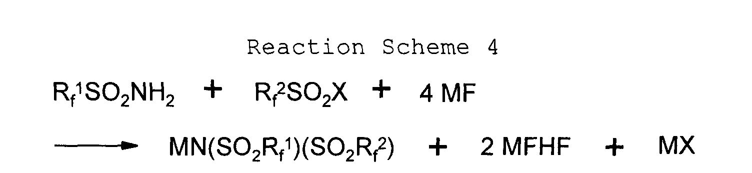
(3) In the case of using an ammonium salt: 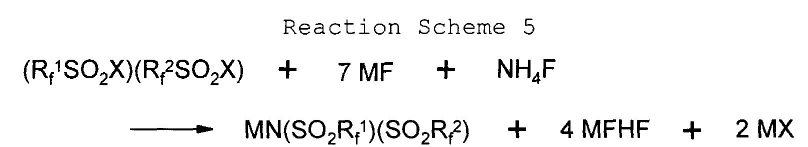 wherein R_{f}¹ and R_{f}² are the same or different. 1 mol of an ammonium salt, 2 mol of at least one of the sulfonyl halogenides represented by the formula (II) R_{f}SO₂X and 7 mol of a fluorine compound represented by the formula (III) MF are introduced into a reactor, and the mixture is reacted in a solvent.

After completion of the reaction, 2 mol of the by-product MX and 4 mol of the by product hydrogendifluoride MFHF are removed by filtration, and then the filtrate is concentrated. The sulfonylimide compound represented by the formula (I) MN(SO₂R_{f}¹) (SO₂R_{f}²) can be thereby produced.

Although MF reacts when its amount is both more and less than the number of moles described above, the reaction efficiency is made lower.

These reactions can occur in a temperature range between about -30°C and 200°C. At a temperature less than the above range, the reaction rate is very low whereas at a temperature exceeding the above range, decomposition of the compounds, solvent and product to be used arises.
A more preferable temperature range is between 0°C and 100°C.

As to the solvent, any solvent may be used without particular limitations as far as it is inert to the reaction materials. For example, ethers such as diethyl ether and tetrahydrofuran, halogenated hydrocarbons such as dichloromethane and dichloroethane, hydrocarbons such as benzene, heptane and hexane and nitriles such as acetonitrile may be used.

In order to produce various sulfonylimide compounds other than those mentioned above, a sulfonylimide compound obtained by these production methods may be made into an acid by using concentrated sulfuric acid and distilling the acid to thereby synthesize a sulfonylimidic acid [HN(SO₂R_{f}¹) (SO₂R_{f}²)]. This acid may be further reacted with a compound selected from hydroxides, oxides, carbonates and acetates of metals corresponding to this acid.

In this case, fluorine compounds represented by the formula (III) MF to be used in the synthesis of a sulfonylimide compound may be used.

### EXAMPLES

The present invention will be described in more detail by way of examples, which, of course, are not intended to be limiting of the present invention.

### Example 1 - KN(SO₂CF₃)₂ and LiN(SO₂CF₃)₂

A flask with four necks was charged with 150 ml of acetonitrile, 23.4 g of potassium fluoride and 20 g of trifluoromethylsulfonylamide, CF₃SO₂NH₂. The reactor was soaked in a 40°C hot water bath, and 25.1 g of trifluoromethylsulfonyl fluoride CF₃SO₂F was introduced with sufficient stirring. The reaction solution was subjected to filtration, and the filtrate was concentrated under reduced pressure to obtain potassium bistrifluoromethylsulfonylimide KN(SO₂CF₃)₂ in an amount of 42.7 g. The yield was 99%.

Next, 42.7 g of this potassium bistrifluoromethylsulfonylimide was added in a flask charged with 60 ml of concentrated sulfuric acid and the mixture was dissolved under heat. Under reduced pressure, 34.6 g of bistrifluoromethylsulfonylimidic acid HN(SO₂CF₃)₂ was distilled by distillation. The yield was 92%.

Then, 34.6 g of the resulting bistrifluoromethylsulfonylimidic acid was dissolved in pure water and reacted with 4.5 g of lithium carbonate. Excess lithium carbonate was removed by filtration and the filtrate was concentrated to obtain 34.6 g of lithium bistrifluoromethylsulfonylimide LiN(SO₂CF₃)₂. The yield was 98%.

### Example 2 - KN(SO₂CF₃)₂

An autoclave made of stainless steel was charged with 200 ml of acetonitrile and 68.3 g of potassium fluoride. The reactor was cooled to -60°C in a dry ice/methanol bath, and 5 g of anhydrous ammonia was introduced.

In succession, 90.0 g of trifluoromethylsulfonyl fluoride CF₃SO₂F was introduced, and the temperature of the mixture was returned to ambient temperature with sufficient stirring. After that, the reactor was soaked in a 40°C hot water bath, and the reaction was completed while stirring sufficiently. The reaction solution was subjected to filtration, and the filtrate was concentrated under reduced pressure to obtain 88.2 g of potassium bistrifluoromethylsulfonylimide KN(SO₂CF₃)₂.
The yield was 95%.

### Example 3 - KN(SO₂CF₃)₂

A flask with four necks was charged with 1 litre of methylene chloride, 10 g of ammonium fluoride and 78.4 g of potassium fluoride. The reactor was soaked in a 40°C hot water bath, and 82.1 g of trifluoromethylsulfonyl fluoride CF₃SO₂F was introduced while stirring sufficiently. The reaction solution was subjected to filtration, and the filtrate was concentrated under reduced pressure to obtain 81.5 g of potassium bistrifluoromethylsulfonylimide KN(SO₂CF₃)₂. The yield was 95%.

### Example 4 - NaN(SO₂C₄F₉)(SO₂CF₃)

A flask was charged with 300 ml of DMF (dimethylformamide), 30 g of perfluorobutylsulfonyl fluoride C₄F₉SO₂F, 15.1 g of trifluoromethylsulfonylamide CF₃SO₂NH₂ and 13 g of sodium fluoride, and the mixture was heated to 100°C and sufficiently stirred to react. The reaction solution was subjected to filtration, and the filtrate was concentrated under reduced pressure to obtain 35.1 g of sodium perfluorobutylsulfonyltrifluoromethylsulfonylimide NaN(SO₂C₄F₉) (SO₂CF₃).
The yield was 78.0%.

### Example 5 - CsN(SO₂C₄F₉)₂

A flask with four necks was charged with 200 ml of acetonitrile, 12 g of perfluorobutylsulfonyl fluoride C₄F₉SO₂F, 15.0 g of cesium fluoride, and 0.73 g of ammonium fluoride, and the mixture was heated to 50°C and sufficiently stirred to react. The reaction solution was subjected to filtration and the filtrate was concentrated under reduced pressure to obtain 12.5 g of cesium bisperfluorobutylsulfonylimide CsN(SO₂C₄F₉)₂. The yield was 89.3%.

### Example 6 - LiN (SO₂CF₃)₂

An autoclave made of stainless steel was charged with 100 ml of dichloromethane, 100 ml of DMF (dimethylformamide) and 30.5 g of lithium fluoride. The reactor was cooled to -60°C in a dry ice/methanol bath and 5 g of anhydrous ammonia was introduced.

In succession, 100 g of trifluoromethylsulfonyl fluoride CF₃SO₂F was introduced, and the temperature of the mixture was returned to ambient temperature with sufficient stirring. After that, the reactor was soaked in a 50°C hot water bath, and the reaction was run while stirring sufficiently. The reaction solution was subjected to filtration, and the filtrate was concentrated under reduced pressure, but only 1.7 g of lithium bistrifluoromethylsulfonylimide LiN(SO₂CF₃)₂. The yield was 2.0%.

Although the amount and percentage yield of the product compound in this case were lower than in the case of the other examples, the reaction may be optimized to improve yield.

### Example 7 - KN(SO₂CF₃)₂ and LiN(SO₂CF₃)₂

A flask with four necks was charged with 150 ml of acetonitrile, 31.2 g of potassium fluoride, and 10 g of trifluoromethylsulfonylamide CF₃SO₂NH₂ were added. The reactor was soaked in a 40°C water bath, and 11.3 g of trifluoromethylsulfonyl chloride CF₃SO₂Cl was introduced while stirring sufficiently. The reaction solution was subjected to filtration, and the filtrate was concentrated under reduced pressure to obtain 21.4 g of potassium bistrifluoromethylsulfonylimide KN(SO₂CF₃)₂. The yield was 96%.

In succession, 21.4 g of potassium bistrifluoromethylsulfonylimide KN(SO₂CF₃)2 was added in a flask that was charged with 30 ml of concentrated sulfuric acid, and the mixture was dissolved under heat. And then, 15.6 g of bistrifluoromethylsulfonylimidic acid HN(SO₂CF₃)₂ was distilled by distillation under reduced pressure. The yield was 83%.

The resulting 15.6 g of bistrifluoromethylsulfonylimidic acid was dissolved in the pure water and reacted with 2.1 g of lithium carbonate. Excess lithium carbonate was subjected to filtration, and the filtrate was concentrated to obtain 15.4 g of lithium bistrifluoromethylsulfonylimide LiN(SO₂CF₃)₂. The yield was 97%.

### Example 8 - KN(SO₂CF₃)₂

A flask with four necks was charged with 200 ml of methylene chloride, 10 g of ammonium fluoride, and 110 g of potassium fluoride. The reactor was soaked in a 40°C water bath, and 91.0 g of trifluoromethylsulfonyl chloride CF₃SO₂Cl was introduced while stirring sufficiently. The reaction solution was subjected to filtration, and the filtrate was concentrated under reduced pressure to obtain 81.0 g of potassium bistrifluoromethylsulfonylimide KN(SO₂CF₃)₂. The yield was 94%.

### Example 9 - CsN(SO₂C₂F₅)(SO₂CF₃)

A flask with four necks was charged with 5 g of ammonium fluoride, 143.6 g of cesium fluoride, and 200 ml of tetrahydrofuran. With sufficiently stirring the reactor, 22.8 g of trifluoromethylsulfonyl chloride CF₃SO₂Cl was introduced, and then 27.3 g of pentafluoroethylsulfonyl fluoride C₂F₅SO₂F was added. The reaction solution was treated in the same way as Example 2 to obtain 62 g of cesium perfluoroethylsulfonyl trifluoromethylsulfonylimide CsN (SO₂C₂F₅) (SO₂CF₃). The yield was 99.2%.

### Example 10 - LiN (SO₂CF₃)₂

In a stainless steel autoclave, 200 ml of methylene chloride, 300 ml of DMF (dimethylformamide), 45.6 g of lithium fluoride, and 99.0 g of trifluoromethylsulfonyl chloride CF₃SO₂Cl were added. The reactor was cooled to - 60°C in a methanol/dry ice bath, and 5 g of anhydrous ammonia was introduced.

The reaction mixture was returned to the room temperature while stirring sufficiently, and then the reactor was soaked in a 80°C water bath, and the reaction was run while stirring sufficiently. Consequently, the reaction solution was treated in the same way as Example 2, but only 0.8 g of lithium bistrifluoromethylsulfonylimide LiN(SO₂CF₃)₂ was obtained. The yield was 0.9%.

Although the amount and percentage yield of the product compound in this case were lower than in the case of the other examples, the reaction may be optimized to improve yield.

### Example 11 - NaN(SO₂C₄F₉)(SO₂CF₃)

In a stainless steel autoclave, 200 ml of methylene chloride, 300 ml of DMF (dimethylformamide), 74.1 g of sodium fluoride, and 49.5 g of trifluoromethylsulfonyl chloride CF₃SO₂Cl were added. The reactor was cooled to - 60°C in a methanol/dry ice bath, and 5 g of anhydrous ammonia was introduced.

Subsequently, 88.8 g of perfluorobuthylsulfonyl fluoride C₄F₉SO₂F was added, and the reaction mixture was returned to room temperature while stirring sufficiently. Then, the reactor was soaked in a 80°C water bath, and the reaction was run while stirring sufficiently. Consequently, the reaction solution was treated in the same way as Example 2, to obtain 18 g of sodium perfluorobuthylsulfonyl trifluoromethylsulfonylimide NaN(SO₂C₄F₉) (SO₂CF₃). The yield was 13.5%.

It is to be noted that the sulfonylimide compounds obtained in the above examples were respectively confirmed by identification using infrared absorption spectroscopy.

As seen from the above description, the production process of the present invention has such an effect that sulfonylimide compounds useful as lithium battery electrolytes and organic synthetic catalysts can be produced industrially, easily, at a low cost, and in an efficient manner.

Furthermore, the production process according to the present invention has such an effect that by reacting a sulfonylamide, a sulfonyl fluoride and a fluorine compound with each other, a sulfonylimide compound useful as lithium battery electrolytes and organic synthetic catalysts can be produced under mild conditions, e.g., in which anhydrous ammonia is not always used, and in one stage.

Also, a specific reactor (autoclave) is not required unlike the case that uses anhydrous ammonia.

## Claims

1. A process for producing a sulfonylimide compound represented by the formula (I):
MN(SO₂R_{f}¹) (SO₂R_{f}²)
wherein:
M represents any one of Li, Na, K and Cs among alkali metals of group Ia in the periodic table; and,
R_{f}¹ and R_{f}², which are the same of different and respectively represent any one of a straight chain or branched compound of a fluoroalkyl, perfluoroalkyl, fluoroallyl or fluoroalkenyl group having 1 to 12 carbon atoms;
the process comprising reacting:
(a) at least one of the sulfonyl halogenides represented by the formula (II):
R_{f}SO₂X
wherein R_{f} represents the same group as R_{f}¹ or R_{f}² in the formula (I); and, X represents either F or Cl among halogen elements of group VIIb of the periodic table; with:
(b) anhydrous ammonia or an ammonium salt; and with:
(c) a fluorine compound represented by the formula (III):
MF
wherein M represents any one of Li, Na, K and Cs among alkali metals of group Ia in the periodic table.

2. A process according to claim 1, wherein step (b) employs an ammonium salt.

3. A process according to claim 1 or 2, wherein an ammonium fluoride or an ammonium hydrogendifluoride is used as the ammonium salt.

4. A process for producing a sulfonylimide compound represented by the formula (I):
MN(SO₂R_{f}¹)(SO₂R_{f}²)
wherein:
M represents any one of Li, Na, K and Cs among alkali metals of group Ia in the periodic table;
R_{f}¹ and R_{f}², which are the same of different, respectively represent any one of a straight chain or branched compound of a fluoroalkyl, perfluoroalkyl, fluoroallyl or fluoroalkenyl group having 1 to 12 carbon atoms;
the process comprising reacting:
(a) a sulfonylamide represented by the formula (IV):
R_{f}SO₂NH₂
wherein R_{f} represents the same group as R_{f}¹ or R_{f}² in the formula (I); with:
(b) at least one of the sulfonyl halogenides represented by the formula (II):
R_{f} SO₂X
wherein R_{f} represents the same group as R_{f}¹ or R_{f}² in the formula (I); and, X represents either F or Cl among halogen elements of group VIIb of the periodic table; and with:
(c) a fluorine compound represented by the formula (III):
MF
wherein M represents any one of Li, Na, K and Cs among alkali metals of group Ia in the periodic table.

5. A process according to any one of claims 1 to 4, wherein X is F.

6. A process according to any one of claims 1 to 5, wherein R_{f} is CF₃, C₂F₅, or C₄F₉.

7. A process according to any one of claims 1 to 6, wherein M is Li, Na, K, or Cs.

8. A process according to any one of claims 1 to 6, wherein M is K.

9. A process according to any one of claims 1 to 7, wherein the compound of formula (I) is LiN(SO₂CF₃)₂, NaN(SO₂CF₃)₂, KN(SO₂CF₃)₂, CsN(SO₂CF₃)₂, LiN(SO₂C₄F₉)₂, NaN(SO₂C₄F₉)₂, KN(SO₂C₄F₉)₂, CsN(SO₂C₄F₉)₂, LiN(SO₂CF₃) (SO₂C₄F₉), NaN(SO₂CF₃) (SO₂C₄F₉), KN(SO₂CF₃) (SO₂C₄F₉), CsN (SO₂CF₃) (SO₂C₂F₅), or CsN(SO₂CF₃) (SO₂C₄F₉).

10. A process according to any one of claims 1 to 8, wherein the compound of formula (I) is LiN(SO₂CF₃)₂, KN(SO₂CF₃)₂, CsN(SO₂C₄F₉)₂, NaN(SO₂C₄F₉)(SO₂CF₃), or CsN(SO₂CF₃)(SO₂C₂F₅).
